# EUROPÄISCHE PATENTANMELDUNG

(11) **EP 2 333 544 A1**
(43) Veröffentlichungstag der Anmeldung: **15.06.2011**
(21) Anmeldenummer: 09178958.6
(22) Anmeldetag: 11.12.2009
(51) Int. Cl.: G01N 33/50

(54) **Sterilisierbare Chemie für Testelemente**

(71) Anmelder: F. Hoffmann-La Roche AG, 4070 Basel (CH); Roche Diagnostics GmbH, 68305 Mannheim (DE)
(72) Erfinder: Horn, Carina Dr., 68647 Biblis (DE); Steinke, Nelli Dr., 67071 Ludwigshafen (DE)
(74) Vertreter: Weiss, Wolfgang

(57) **Zusammenfassung**

Die vorliegende Erfindung betrifft ein diagnostisches Element sowie ein Verfahren zu dessen Herstellung.

## Beschreibung

Die vorliegende Erfindung betrifft ein diagnostisches Element sowie ein Verfahren zu dessen Herstellung.

Diagnostische Elemente sind wichtige Bestandteile klinisch relevanter Analyseverfahren. Hierbei steht die Messung von Analyten, z. B. Metaboliten oder Substraten, im Vordergrund, welche beispielsweise mit Hilfe eines für den Analyten spezifischen Enzyms direkt oder indirekt bestimmt werden. Die Analyten werden hierbei mit Hilfe eines Enzym-Coenzym-Komplexes umgesetzt und anschließend quantifiziert. Dabei wird der zu bestimmende Analyt mit einem geeigneten Enzym, einem Coenzym und gegenenfalls einem Mediator in Kontakt gebracht, wobei das Coenzym durch die enzymatische Reaktion physikochemisch verändert, z. B. oxidiert bzw. reduziert, wird. Sofern zusätzlich ein Mediator zum Einsatz gelangt, überträgt dieser die bei Umsetzung des Analyten freigesetzten Elektronen vom reduzierten Coenzym üblicherweise auf einen optischen Indikator oder die leitfähigen Bestandteile einer Elektrode, so dass der Vorgang beispielsweise photometrisch oder elektrochemisch erfasst werden kann. Eine Kalibrierung liefert einen direkten Zusammenhang des Messwerts mit der Konzentration des zu bestimmenden Analyten.

Die Sterilisierbarkeit derartiger Testelemente ist hinsichtlich eines Einsatzes als diagnostisches Mittel von immenser Bedeutung. Aus dem Stand der Technik bekannte diagnostische Elemente, welche beispielsweise zur Blutglucose-Bestimmung eingesetzt werden, besitzen hierbei den Nachteil, dass diese in Bezug auf ionisierende Strahlung oder andere herkömmlich zur Sterilisation verwendete physikalische oder chemische Mittel eine geringe Stabilität besitzen und nach Sterilisation eine signifikante Schädigung insbesondere des Enzymsystems aufweisen. So zeigen derzeit im Handel erhältliche Testelemente, wie beispielsweise Accu-Check Active^{®}, Accu-Check Aviva^{®} oder die in EP 1 430 831 A1 beschriebenen Biosensoren, welche ein Pyrrolochinolinchinon (PQQ)-abhängiges Enzym und einen der Elektronenübertragung dienenden Mediator enthalten, nach Sterilisation mit ionisierender Strahlung einen signifikanten Verlust an Enzymaktivität.

Eine bekannte Maßnahme, die zur Kompensation der durch Sterilisation hervorgerufenen Schädigung des Enzymsystems biochemischer Testelemente eingesetzt wird, besteht in einer Überdosierung des Enzymsystems. EP 1 293 574 B1 offenbart elektrochemische Sensoren, welche eine PQQ-abhängige Glucose-Dehydrogenase in Kombination mit einem Phenothiazin-Mediator umfassen, wobei die PQQ-abhängige Glucose-Dehydrogenase in einer Konzentration von 20 Enzymeinheiten zur Anwendung gelangt. Nach Sterilisation unter Verwendung von Elektronenstrahlung (Dosis 25 kGy) können in Abhängigkeit von der Formulierung des Enzymsystems Sensoren erhalten werden, welche aufgrund der Überdosierung des Enzymsystems trotz sterilisationsbedingten Verlusten eine hohe Absolutmenge an funktionellem Enzym aufweisen.

Die Überdosierung des Enzymsystems in biochemischen Testelementen ist indessen mit Nachteilen verbunden. Ungeachtet der Tatsache, dass eine Überdosierung von Komponenten unwirtschaftlich ist und bei der Herstellung von Verbrauchsartikeln im großindustriellen Maßstab signifikant höhere Produktionskosten verursacht, können insbesondere hohe Konzentrationen an Enzym Probleme in Bezug auf Löslichkeit oder/und Viskosität nach sich ziehen und die Aufbringung des Enzyms auf einen geeigneten Träger erschweren.

Die der vorliegenden Erfindung zugrunde liegende Aufgabe bestand somit darin, ein stabiles biochemisches Testelement, insbesondere zur Bestimmung von Glucose, bereitzustellen, bei welchem die Nachteile des Standes der Technik zumindest teilweise beseitigt sind. Insbesondere sollte das Testelement nach Sterilisation und ohne Überdosierung des Enzymsystems einen hohen Anteil an aktivem Enzym bzw. an aktivem Coenzym aufweisen und eine gute Performance gewährleisten.

Diese Aufgabe wird erfindungsgemäß gelöst durch ein diagnostisches Element, umfassend ein chemisches Nachweisreagenz, welches zumindest eine gegenüber ionisierender Strahlung empfindliche Komponente umfasst, wobei das diagnostische Element einer Sterilisation unterzogen worden ist und die gegenüber ionisierender Strahlung empfindliche Komponente nach Sterilisation zu einem Anteil von ≥ 80%, bezogen auf die Gesamtmenge der Komponente in dem diagnostischen Element vor der Sterilisation, in funktioneller Form vorliegt.

Überraschenderweise wurde im Rahmen der vorliegenden Erfindung festgestellt, dass diagnostische Elemente wie beispielsweise Testbänder oder Teststreifen, welche ein chemisches Nachweisreagenz mit mindestens einer gegenüber ionisierender Strahlung empfindlichen Komponente umfassen, nach Sterilisation keine oder lediglich eine geringe Schädigung jener Komponente zeigen.

Das in den erfindungsgemäßen diagnostischen Elementen eingesetzte chemische Nachweiseragenz kann grundsätzlich beliebige Komponenten umfassen, welche zur Bestimmung eines Analyten, beispielsweise unter Verwendung optischer oder elektrochemischer Mittel, geeignet sind. Beispiele für derartige Komponenten sind dem Fachmann bekannt und umfassen insbesondere Polypeptide, Coenzyme, optische Indikatoren, Mediatoren sowie Hilfs- oder/und Zusatzstoffe, sind jedoch nicht auf diese beschränkt. Das chemische Nachweisreagenz kann dabei in beliebiger Form ausgebildet sein, ist jedoch vorzugsweise Bestandteil mindestens einer Reagenzienschicht, welche auf einen geeigneten Träger aufgebracht ist und gegebenenfalls gleichzeitig zum Nachweis des Analyten verwendet werden kann.

Bei der gegenüber ionisierender Strahlung empfindlichen Komponente kann es sich um eine beliebige Komponente des chemischen Nachweisreagenzes handeln, welche zur Diagnostik eines zu bestimmenden Analyten benötigt wird, d. h. direkt oder indirekt an der physikochemischen Umsetzung des Analyten beteiligt ist, wobei der Ausdruck "gegenüber ionisierender Strahlung empfindlich", wie er hierin verwendet wird, bedeutet, dass die Komponente bei den jeweiligen Umgebungsbedingungen, d. h. bei dem jeweils vorherrschenden Druck, der jeweils vorherrschenden Temperatur und der jeweils vorherrschenden relativen Luftfeuchtigkeit, durch ionisierende Strahlung physikochemisch verändert oder/und in ihrer Funktion beeinträchtigt werden kann. Insofern ist es erfindungsgemäß möglich, dass die gegenüber ionisierender Strahlung empfindliche Komponente nach Sterilisation des diagnostischen Elements auch zu einem Anteil von 100%, bezogen auf die Gesamtmenge der Komponente in dem diagnostischen Element vor der Sterilisation, in funktioneller Form vorliegt, wobei vorstehende Stabilitätskriterien jeweils für das gesamte diagnostische Element gelten.

Bevorzugt handelt es sich bei der gegenüber ionisierender Strahlung empfindlichen Komponente um ein Polypeptid, ein Coenzym, einen optischen Indikator, oder eine Kombination hiervon, wobei Polypeptide besonders bevorzugt sind. Als Polypeptid kommt hierbei jedes beliebige Polypeptid in Frage, welches den jeweiligen Anforderungen entspricht und vom Fachmann als geeignet erachtet wird. Vorzugsweise ist das Polypeptid ein Enzym, insbesondere ein Coenzym-abhängiges Enzym. Beispiele für derartige Enzyme umfassen u.a. Dehydrogenasen, Oxidasen, wie z. B. Glucoseoxidase (EC 1.1.3.4) oder Cholesterinoxidase (EC 1.1.3.6), Aminotransferasen, wie z. B. Aspartat- oder Alanin-Aminotransferase, 5'-Nukleotidase, Creatin-Kinase und Diaphorase (EC 1.6.99.2).

In einer stärker bevorzugten Ausführungsform gelangt als Enzym eine Nikotinamid-Adenin-Dinukleotid (NAD/NADH)- oder Nikotinamid-Adenin-Dinukleotidphosphat (NADP/NADPH)-abhängige Dehydrogenase zur Anwendung, wobei das Enzym insbesondere aus der Gruppe bestehend aus einer Alkohol-Dehydrogenase (EC 1.1.1.1; EC 1.1.1.2), einer L-Aminosäure-Dehydrogenase (EC 1.4.1.5), einer Glucose-Dehydrogenase (EC 1.1.1.47), einer Glucose-6-phosphat-Dehydrogenase (EC 1.1.1.49), einer Glycerin-Dehydrogenase (E.C.1.1.1.6), einer 3-Hydroxybutyrat-Dehydrogenase (EC 1.1.1.30), einer Lactat-Dehydrogenase (EC 1.1.1.27; 1.1.1.28), einer Malat-Dehydrogenase (EC 1.1.1.37) und einer Sorbitol-Dehydrogenase ausgewählt wird. Am stärksten bevorzugt ist das Enzym eine Glucose-Dehydrogenase (EC 1.1.1.47) oder eine Glucose-6-phosphat-Dehydrogenase (EC 1.1.1.49).

Die gegenüber ionisierender Strahlung empfindliche Komponente kann weiterhin ein Coenzym sein. Die vorliegende Erfindung sieht grundsätzlich den Einsatz beliebiger Coenzyme vor; vorzugsweise wird als Coenzym jedoch eine NAD(P)/NAD(P)H-Verbindung eingesetzt. Der Begriff NAD(P)/NAD(P)H-Verbindung, wie er im Rahmen der vorliegenden Anmeldung verwendet wird, umfasst sowohl natürlich vorkommende NAD(P)/NAD(P)H-Verbindungen, wie beispielsweise Nicotinamid-Adenin-Dinukleotid (NAD/NADH) und Nikotinamid-Adenin-Dinukleotidphosphat (NADP/NADPH), als auch artifizielle NAD(P)/NAD(P)H-Verbindungen, welche durch chemische Modifizierung natürlicher NAD(P)/NAD(P)H-Verbindungen erhalten werden können.

In einer Ausführungsform der Erfindung handelt es sich bei dem Coenzym um ein stabilisiertes Coenzym. Ein stabilisiertes Coenzym im Sinne der vorliegenden Erfindung ist ein gegenüber dem nativen Coenzym chemisch verändertes Coenzym, welches bei Atmosphärendruck eine im Vergleich zum nativen Coenzym höhere Stabilität gegenüber Feuchtigkeit, Temperaturen insbesondere im Bereich von 0°C bis 50°C, Säuren und Basen insbesondere im Bereich von pH 4 bis pH 10, oder/und Nukleophilen wie beispielsweise Alkoholen oder Aminen, aufweist und insofern unter identischen Umgebungsbedingungen über einen längeren Zeitraum als das native Coenzym seine Wirkung entfalten kann. Vorzugsweise weist das stabilisierte Coenzym eine im Vergleich zum nativen Coenzym höhere hydrolytische Stabilität auf, wobei eine vollständige Hydrolysestabilität unter Testbedingungen besonders bevorzugt ist. Im Vergleich zum nativen Coenzym kann das stabilisierte Coenzym eine verringerte oder erhöhte Bindungskonstante für das Enzym aufweisen, beispielsweise eine um den Faktor von 2 oder mehr verringerte oder erhöhte Bindungskonstante.

Bevorzugte Beispiele für stabilisierte Coenzyme im Sinne der vorliegenden Erfindung sind stabilisierte NAD(P)/NAD(P)H-Verbindungen, d. h. chemische Derivate von nativem Nikotinamid-Adenin-Dinukleotid (NAD/NADH) bzw. Nikotinamid-Adenin-Dinukleotidphosphat (NADP/NADPH), oder die Verbindung der Formel (I)

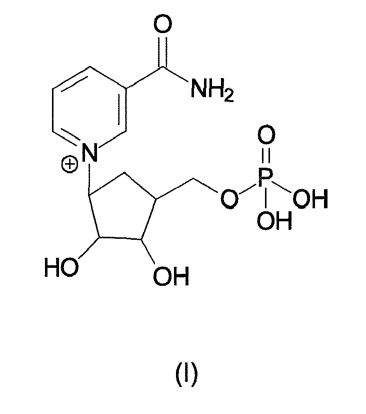

Sofern es sich bei dem stabilisierten Coenzym um eine stabilisierte NAD(P)/NAD(P)H-Verbindung handelt, umfasst die stabilisierte NAD(P)/NAD(P)H-Verbindung vorzugsweise einen 3-Pyridincarbonyl- oder einen 3-Pyridinthiocarbonyl-Rest, welcher ohne glykosidische Bindung über einen linearen oder cyclischen organischen Rest, insbesondere über einen cyclischen organischen Rest, mit einem phosphorhaltigen Rest, wie beispielsweise einem Phosphatrest, verknüpft ist.

Besonders bevorzugt wird die stabilisierte NAD(P)/NAD(P)H-Verbindung aus Verbindungen der allgemeinen Formel (II) ausgewählt: 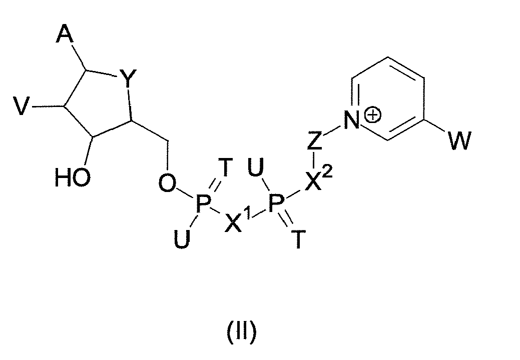 worin
- A =: Adenin oder ein Analog davon,
- T =: jeweils unabhängig O, S,
- U =: jeweils unabhängig OH, SH, BH₃⁻, BCNH₂⁻,
- V =: jeweils unabhängig OH oder eine Phosphatgruppe, oder zwei Gruppen, die eine cyclische Phosphatgruppe bilden;
- W =: COOR, CON(R)₂, COR, CSN(R)₂ mit R = jeweils unabhängig H oder C₁-C₂-Alkyl,
- X¹, X² =: jeweils unabhängig O, CH₂, CHCH₃, C(CH₃)₂, NH, NCH₃,
- Y =: NH, S, O, CH₂,
- Z =: ein linearer oder cyclischer organischer Rest ist, mit der Maßgabe, dass Z und der Pyridin-Rest nicht durch eine glycosidische Verbindung verknüpft sind, oder ein Salz oder gegebenenfalls eine reduzierte Form davon.

In den Verbindungen der Formel (II) ist Z vorzugsweise ein linearer Rest mit 4-6 C-Atomen, vorzugsweise mit 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, CI, CH₃.

Besonders bevorzugt ist Z ein gesättiger oder ungesättigter carbocyclischer oder heterocyclischer Fünfring, insbesondere eine Verbindung der allgemeinen Formel (III), 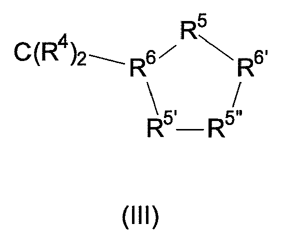 wobei zwischen R^{5'} und R^{5"}eine Einfach- oder Doppelbindung vorliegen kann, mit
- R⁴ =: jeweils unabhängig H, F, Cl, CH₃,
- R⁵ =: CR⁴₂,
- R^{5'} =: O, S, NH, NC₁-C₂-Alkyl, CR⁴₂, CHOH, CHOCH₃, und R^{5"} = CR⁴₂, CHOH, CHOCH₃, wenn zwischen R^{5'} und R^{5"} eine Einfachbindung vorliegt,
- R^{5'} = R^{5"} = CR⁴,: wenn zwischen R^{5'} und R^{5"} eine Doppelbindung vorliegt, und
- R⁶, R^{6'} =: jeweils unabhängig CH oder CCH₃.

In einer bevorzugten Ausführungsform enthalten die erfindungsgemäßen Verbindungen Adenin oder Adenin-Analoga, wie z.B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁₋₆-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxydesoxyribose, 2'-Fluorodesoxyribose, Hexitol, Altritol bzw. polycyclische Analoga, wie Bicyclo-, LNA- und Tricyclo-Zucker enthalten.

Insbesondere können in den Verbindungen der Formel (II) auch (Di-)-Phosphatsauerstoffe isotronisch ersetzt sein, wie z.B. O⁻ durch S⁻ bzw. BH₃⁻, O durch NH, NCH₃ bzw. CH₂ und =O durch =S. In den erfindungsgemäßen Verbindungen der Formel (II) ist W vorzugsweise CONH₂ oder COCH_{3.}

In den Gruppen der Formel (III) ist R⁵ vorzugsweise CH₂. Weiterhin ist bevorzugt, dass R^{5'} ausgewählt ist aus CH₂, CHOH und NH. In einer besonders bevorzugten Ausführungsform sind R^{5'} und R^{5"} jeweils CHOH. In noch einer weiteren bevorzugten Ausführungsform ist R^{5'} NH und R^{5"} CH₂. Stärker bevorzugt ist eine Verbindung der Formel (III), in welcher R⁴ = H ist, R⁵ = CH₂ ist, R^{5'} = R^{5"} = CHOH ist, und R⁶ = R^{6'} = CH ist.

In der am stärksten bevorzugten Ausführungsform handelt es sich bei der stabilisierten NAD(P)/NAD(P)H-Verbindung um die literaturbekannte Verbindung carbaNAD (J.T. Slama, Biochemistry (1988), 27, 183 und Biochemistry (1989), 28, 7688). Andere stabile Coenzyme, welche erfindungsgemäß Anwendung finden können, sind in WO 98/33936, WO 01/49247, WO 2007/012494, US 5,801,006, US 11/460,366 und der Publikation Blackburn et al. (Chem. Comm. (1996), 2765) beschrieben, auf deren Offenbarung hiermit ausdrücklich Bezug genommen wird.

Alternativ kann die gegenüber ionisierender Strahlung empfindliche Komponente auch ein optischer Indikator sein. Als optischer Indikator kann eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden optischen, insbesondere photometrischen oder fluorimetrischen, oder elektrochemischen Verfahrens erfolgen. Vorzugsweise werden Heteropolysäuren, und insbesondere 2,18-Phosphormolybdänsäure, als optische Indikatoren verwendet, die zum entsprechenden Heteropolyblau reduziert werden. Ferner können auch Chinone wie beispielsweise Resazurin, Dichlorphenolindophenol oder/und Tetrazoliumsalze als optische Indikatoren eingesetzt werden. Tetrazoliumsalze, welche für die Zwecke der vorliegenden Erfindung besonders geeignet sind, umfassen beispielsweise die kommerziell erhältlichen Produkte WST-3, WST-4 und WST-5 (alle Fa. Dojindo), sind jedoch nicht auf diese beschränkt.

Das diagnostische Element kann ein beliebiges Element sein, welches von der den Analyten enthaltenden Probe benetzt werden kann. So kann das diagnostische Element die gegenüber ionisierender Strahlung empfindliche(n) Komponente(n), wie etwa ein Polypeptid oder ein Coenzym, beispielsweise in einer oder mehreren Reagenzienschichten enthalten, welche gegebenenfalls weitere Reagenzien, die der qualtitativen oder/und quantitativen Bestimmung des Analyten dienen, beinhalten. Beispiele für derartige Reagenzien umfassen u.a. Mediatoren sowie geeignete Hilfs- oder/und Zusatzstoffe.

Der Begriff "Mediator", wie er im Rahmen dieser Anmeldung verwendet wird, bezeichnet eine chemische Verbindung, welche mit dem durch Umsetzung mit dem Analyten erhaltenen, reduzierten Coenzym reagiert und eine Übertragung von Elektronen auf einen geeigneten optischen Indikator oder ein optisches Indikatorsystem oder auf elektrochemische Elektroden ermöglicht. Als Mediatoren kommen erfindungsgemäß u.a. Nitrosoaniline, wie beispielsweise [(4-Nitrosophenyl)imino]dimethanol-Hydrochlorid, Chinone, wie beispielsweise Phenanthrenchinone, Phenanthrolinchinone oder Benzo[h]-chinolinchinone, Phenazine, wie beispielsweise 1-(3-Carboxypropoxy)-5-ethylphenazinium-trifluormethansulfonat, oder/und Diaphorase (EC 1.6.99.2) in Frage._Bevorzugt enthalten die diagnostischen Elemente der vorliegenden Erfindung allerdings keinen Mediator, was den Vorteil besitzt, dass Nebenreaktionen des Mediators vermieden werden und damit die Stabilität des diagnostischen Elements auf Dauer nicht beeinträchtigt wird.

Die diagnostischen Elemente der vorliegenden Erfindung weisen in Abhängigkeit von der verwendeten Sterilisationsmethode die gegenüber ionisierender Strahlung empfindliche Komponente nach Sterilisation in einem Anteil von ≥ 80%, bevorzugt in einem Anteil von ≥ 90%, bezogen auf die Gesamtmenge der Komponente in dem diagnostischen Element vor der Sterilisation, in funktioneller Form auf, wobei ein Anteil von 100%, wie vorstehend beschrieben, definitionsgemäß ebenfalls umfasst ist. Der Begriff "in funktioneller Form", wie er im Rahmen der vorliegenden Anmeldung verwendet wird, bedeutet hierbei, dass die Komponente in chemisch aktiver Form vorliegt und die ihr zugedachte Funktion in dem diagnostischen Element erfüllen kann. Der Begriff "in nicht-funktioneller Form" bedeutet demgegenüber, dass die Komponente in chemisch inaktiver Form vorliegt oder ihre vorgesehene Funktion trotz Vorliegens in einer chemisch aktiven Form, welche sich von der zur Ausübung der gewünschten Funktion erforderlichen Form unterscheidet, nicht erfüllt.

Erfindungsgemäß liegen somit mindestens 80% der vor Sterilisation in dem diagnostischen Element vorhandenen Moleküle einer gegenüber ionisierender Strahlung empfindlichen Komponente, d. h. funktionelle plus nicht-funktionelle Moleküle, nach Sterilisation in aktiver Form vor und können insofern, gegebenenfalls zusammen mit anderen Komponenten des chemischen Nachweisreagenzes, die gewünschte Umsetzung des zu bestimmenden Analyten bewirken. Die Stabilität der erfindungsgemäßen diagnostischen Elemente gegenüber ionisierender Strahlung hat insofern den Vorteil, dass eine Überdosierung einzelner Komponenten des chemischen Nachweisreagenzes (z. B. eines Enzyms) in Bezug auf andere Komponenten des chemischen Nachweisreagenzes, welche einer Kompensation des sterilisationsbedingten Verlusts an funktionellen Molekülen der jeweiligen Komponente dient, vermieden werden kann.

In einer bevorzugten Ausführungsform umfassen die diagnostischen Elemente der vorliegenden Erfindung neben der mindestens einen gegenüber ionisierender Strahlung empfindlichen Komponente weiterhin ein Element zur Probennahme (Probennahmeelement), welches integriert oder separat vorliegen kann. Unter einem integrierten Probennahmeelement im Sinne der vorliegenden Erfindung ist eine Vorrichtung zu verstehen, welche physikalisch mit dem diagnostischen Element verbunden ist und die aufgenommene Probe über geeignete Mittel, wie beispielsweise einen Kapillarkanal, direkt auf das diagnostische Element transferieren kann.

Im Gegensatz hierzu ist ein separates Probennahmeelement als eine vom diagnostischen Element getrennt vorliegende Probennahmevorrichtung definiert, welche keine physikalische Verbindung mit dem diagnostischen Element aufweist. In diesem Fall kann ein Transfer der Probe auf das erfindungsgemäße diagnostische Element beispielsweise im Anschluss an eine Remagazinierung der Probennahmevorrichtung erfolgen, wobei das diagnostische Element in dem Magazin positioniert ist.

Als Probennahmeelement kann ein beliebiges Element verwendet werden, welches eine Probe des Analyten aufzunehmen vermag und einen nachfolgenden Transfer der Probe auf das diagnostische Element, beispielsweise unter Ausnutzung von Kapillareffekten, ermöglicht. Als vorteilhaft hat sich in diesem Zusammenhang insbesondere die Verwendung eines Nadelelements erwiesen, welches vorzugsweise einen Kapillarkanal zur Aufnahme der Probe umfasst und aus einem beliebigen Material, vorzugsweise jedoch aus einem sterilisierbaren Material wie beispielsweise Metall oder Kunststoff, besteht.

Erfindungsgemäß ist es bevorzugt, dass das diagnostische Element zusammen mit dem Probennahmeelement, insbesondere mit einem Nadelelement, in einem Vorratsbehälter gelagert ist. Der Vorratsbehälter kann grundsätzlich aus einem beliebigen Material bestehen, welches dem Fachmann für die Zwecke einer Lagerung diagnostischer Elemente geeignet erscheint. Als bevorzugt sind in diesem Zusammenhang Vorratsbehälter anzusehen, welche teilweise oder vollständig aus Kunststoff bestehen, wobei insbesondere Kunststoffe auf Basis von Polyamid, Polycarbonat, Polyester, Polyethylen und Polypropylen zur Anwendung gelangen.

Besonders bevorzugt handelt es sich bei dem Vorratsbehälter um ein Magazin, welches mehrere erfindungsgemäße diagnostische Elemente umfasst. Der Begriff "mehrere", wie er hierin verwendet wird, bedeutet dabei jegliche Zahl > 1, wobei bevorzugt mindestens 10, besonders bevorzugt mindestens 25 diagnostische Elemente in dem Magazin gelagert werden können. Das Magazin kann in beliebiger Art und Weise ausgestaltet sein, umfasst jedoch insbesondere Blistermagazine, Scheibenmagazine und Trommelmagazine, wie sie beispielsweise in EP 0 951 939 A2 und WO 2005/104948 A1 beschrieben sind und auf deren Offenbarung hiermit ausdrücklich Bezug genommen wird.

Die Herstellung der hierin beschriebenen diagnostischen Elemente umfasst eine Sterilisation, wobei ein diagnostisches Element, umfassend ein chemisches Nachweisreagenz mit mindestens einer gegenüber ionisierender Strahlung empfindlichen Komponente sowie gegebenenfalls ein integriertes oder separates Probennahmeelement, bereitgestellt und anschließend einer Sterilisation unterzogen wird. Erfindungsgemäß kann das diagnostische Element vor oder nach dem Sterilisieren in einen geeigneten Vorratsbehälter, wie beispielsweise in ein Magazin, eingebracht werden, wobei ein entsprechendes Einbringen vor Durchführen der Sterilisation als bevorzugt anzusehen ist.

Sofern das diagnostische Element vor dem Sterilisieren in einen Vorratsbehälter eingebracht wird, so besteht grundsätzlich die Möglichkeit, dass der Vorratsbehälter vor oder nach Sterilisieren des diagnostischen Elements verschlossen wird, wobei ein Verschließen vor Durchführung der Sterilisation insbesondere aus hygienebedingten Gründen zu bevorzugen ist. In einer besonders bevorzugten Ausführungsform der Erfindung ist es insofern vorgesehen, dass die Herstellung des diagnostischen Elements das Einbringen des diagnostischen Elements in ein Magazin, Verschließen des Magazins und anschließendes Sterilisieren des diagnostischen Elements in dem verschlossenen Magazin umfasst, wobei das diagnostische Element ein vorzugsweise separates Nadelelement umfasst.

Die Sterilisation selbst kann auf unterschiedliche Art und Weise erfolgen und umfasst beispielsweise chemische Sterilisation, Sterilisation durch Erhitzen und Sterilisation mittels ionisierender Strahlung. Erfindungsgemäß hat es sich als vorteilhaft erwiesen, die Sterilisation mittels ionisierender Strahlung durchzuführen, wobei die ionisierende Strahlung bevorzugt Elektronenstrahlung oder/und Gammastrahlung, besonders bevorzugt Elektronenstrahlung, umfasst.

Die Dosis der zur Sterilisation verwendeten ionisierenden Strahlung kann vom Fachmann entsprechend den jeweiligen Anforderungen ausgewählt werden. In einer bevorzugten Ausführungsform weist die zur Sterilisation des diagnostischen Elements verwendete Elektronenstrahlung indessen eine Dosis im Bereich von 15-35 kGy, insbesondere im Bereich von 20-30 kGy, auf, während im Falle der Verwendung von Gammastrahlung eine Dosis im Bereich von 15-35 kGy, insbesondere im Bereich von 20-30 kGy, angewendet wird.

Das mittels obigem Verfahren sterilisierte diagnostische Element wird, gegebenenfalls zusammen mit dem integrierten oder separaten Probennahmeelement, im Anschluss an die Sterilisation vorzugsweise steril verpackt. Die sterile Verpackung ermöglicht es, das erfindungsgemäße diagnostische Element bis zu seinem späteren Gebrauch steril zu halten, ohne dass es einer erneuten Sterilisation bedarf. Besonders bevorzugt handelt es sich bei dem diagnostischen Element der vorliegenden Erfindung insofern um einen Einwegartikel, welcher nach Gebrauch infolge Verlust der Sterilität nicht erneut verwendet wird.

Die hierein offenbarten diagnostischen Elemente umfassen grundsätzlich jede dem Fachmann geläufige physikalische Form, welche für die

Bestimmung des Vorhandenseins oder/und der Menge eines Analyten in einer Probe geeignet ist. Die diagnostischen Elemente umfassen jeweils mindestens einen Testbereich, welcher mit einer den Analyten enthaltenden Probe in Kontakt gebracht werden kann und unter Verwendung geeigneter Mittel eine qualitative oder/und quantitative Bestimmung des Analyten ermöglicht. In einer bevorzugten Ausführungsform der Erfindung ist das diagnostische Element derart ausgebildet, dass es bei Anwesenheit des zu bestimmenden Analyten ein optisch oder elektrochemisch detektierbares Signal erzeugt, womit unter Verwendung optischer Methoden, wie z. B. Photometrie oder Fluorimetrie, oder elektrochemischer Techniken eine qualitative oder/und quantitative Bestimmung des Analyten ermöglicht wird. Beispiele für diagnostische Elemente im Sinne der vorliegenden Erfindung umfassen insbesondere Testelemente mit integriertem Nadelelement, Testbänder, Teststreifen sowie die in WO 2005/084530 A2 beschriebenen diagnostischen Elemente, auf die der Analyt beispielsweise in Form einer wässrigen oder nichtwässrigen Lösung aufgebracht werden kann. Auf die Offenbarung vorstehender internationaler Anmeldung wird hiermit ausdrücklich Bezug genommen.

Die erfindungsgemäßen diagnostischen Elemente können zur Bestimmung einer beliebigen biologischen oder chemischen Substanz verwendet werden, welche photochemisch oder elektrochemisch nachweisbar ist. Vorzugsweise wird der Analyt aus der Gruppe bestehend aus Äpfelsäure, Alkohol, Ammonium, Ascorbinsäure, Cholesterin, Cystein, Glucose, Glutathion, Glycerin, Harnstoff, 3-Hydroxybutyrat, Milchsäure, 5'-Nukleotidase, Peptiden, Pyruvat, Salicylat und Triglyceriden ausgewählt, wobei Glucose besonders bevorzugt ist. Der Analyt kann hierbei aus einer beliebigen Quelle stammen, ist jedoch bevorzugt in einem Körperfluid, umfassend, jedoch nicht beschränkt auf, Vollblut, Plasma, Serum, Lymphflüssigkeit, Gallenflüssigkeit, Cerebrospinalflüssigkeit, extrazelluläre Gewebeflüssigkeit, Harn, sowie Drüsensekrete wie beispielsweise Speichel oder Schweiß, enthalten. Bevorzugt wird mittels der hierin beschriebenen diagnostischen Elemente das Vorhandensein oder/und die Menge eines Analyten in einer Probe aus Vollblut, Plasma, Serum oder extrazellulärer Gewebeflüssigkeit bestimmt.

Die qualitative oder/und quantitative Bestimmung des Analyten kann auf beliebige Art und Weise erfolgen. Hierzu können grundsätzlich alle aus dem Stand der Technik bekannten Methoden zum Nachweis enzymatischer Reaktionen eingesetzt werden, welche ein messbares Signal erzeugen, das manuell oder unter Verwendung geeigneter Mittel ausgewertet bzw. ausgelesen werden kann. Im Rahmen der vorliegenden Erfindung gelangen bevorzugt optische Nachweismethoden, welche beispielsweise die Messung von Absorption, Fluoreszenz, Circulardichroismus (CD), optische Rotationsdispersion (ORD) oder/und Refraktometrie umfassen, zur Anwendung, wobei der Nachweis des Analyten besonders bevorzugt photometrisch oder fluorometrisch, beispielsweise indirekt über eine fluorometrisch detektierbare Veränderung des Coenzyms, erfolgt. Alternativ kann der Analyt auch elektrochemisch nachgewiesen werden, wobei ein elektrisches Signal, wie beispielsweise elektrischer Strom, Spannung oder/und Widerstand, detektiert wird.

In einem weiteren Aspekt betrifft die Erfindung ein Verfahren zur Herstellung eines diagnostischen Elements, umfassend die Schritte:
(a) Bereitstellen eines diagnostischen Elements, umfassend ein chemisches Nachweisreagenz, welches zumindest eine gegenüber ionisierender Strahlung empfindliche Komponente umfasst, wobei die mindestens eine gegenüber ionisierender Strahlung empfindliche Komponente eine Nikotinamid-Adenin-Dinukleotid (NAD/NADH)- oder Nikotinamid-Adenin-Dinukleotidphosphat (NADP/NADPH)-abhängige Dehydrogenase ist, und
(b) Sterilisieren des diagnostischen Elements mit ionisierender Strahlung.

Die Erfindung soll durch die nachfolgenden Figuren und Beispiele näher erläutert werden:

### Beschreibung der Figuren

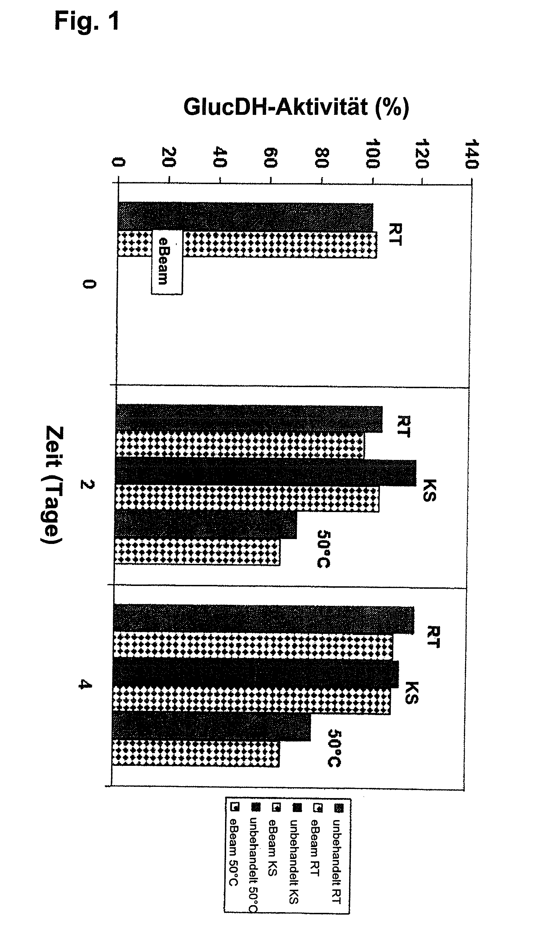

- **Figur 1**:: Aktivität von Glucose-Dehydrogenase (GlucDH) in einem diagnostischen Element, umfassend Glucose-Dehydrogenase und Nikotinamid-Adenin-Dinukleotid, vor und nach Sterilisation mit Elektronenstrahlung (eBeam; Dosis 25 kGy). Die Bestimmung erfolgte unmittelbar nach Herstellung (0 Tage) sowie 2 bzw. 4 Tage nach Lagerung des diagnostischen Elements bei 5°C (KS), Raumtemperatur (RT) bzw. 50°C.

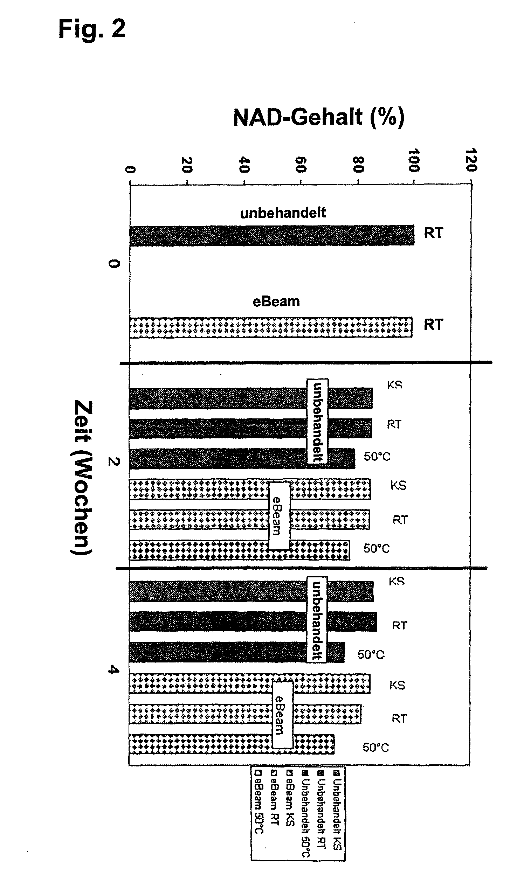

- **Figur 2**:: Gehalt an Nikotinamid-Adenin-Dinukleotid (NAD) in einem diagnostischen Element, umfassend Glucose-Dehydrogenase und Nikotinamid-Adenin-Dinukleotid, vor und nach Sterilisation mit Elektronenstrahlung (eBeam; Dosis 25 kGy). Die Bestimmung erfolgte unmittelbar nach Herstellung (0 Tage) sowie 2 bzw. 4 Wochen nach Lagerung des diagnostischen Elements bei 5°C (KS), Raumtemperatur (RT) bzw.50°C.

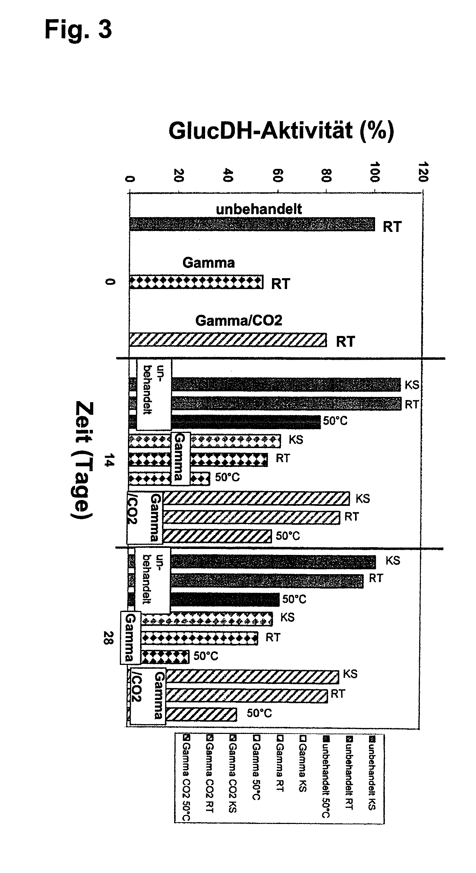

- **Figur 3**:: Aktivität von Glucose-Dehydrogenase (GlucDH) in einem diagnostischen Element, umfassend Glucose-Dehydrogenase und Nikotinamid-Adenin-Dinukleotid, vor und nach Sterilisation mit Gammastrahlung (Gamma; Dosis 25 kGy) bzw. Gammastrahlung in Gegenwart von Kohlendioxid (Gamma/CO₂; Dosis 25 kGy). Die Bestimmung erfolgte unmittelbar nach Herstellung (0 Tage) sowie 2 bzw. 4 Wochen nach Lagerung des diagnostischen Elements bei 5°C, Raumtemperatur (RT) bzw. 50°C.

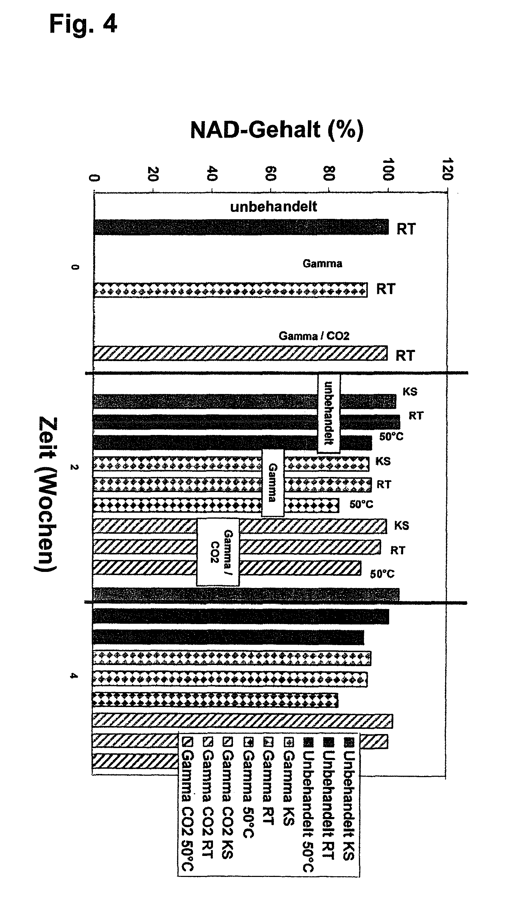

- **Figur 4**:: Gehalt an Nikotinamid-Adenin-Dinukleotid (NAD) in einem diagnostischen Element, umfassend Glucose-Dehydrogenase und Nikotinamid-Adenin-Dinukleotid, vor und nach Sterilisation mit Gammastrahlung (Gamma; Dosis 25 kGy) bzw. Gammastrahlung in Gegenwart von Kohlendioxid (Gamma/CO₂; Dosis 25 kGy). Die Bestimmung erfolgte unmittelbar nach Herstellung (0 Tage) sowie 2 bzw. 4 Wochen nach Lagerung des diagnostischen Elements bei 5°C, Raumtemperatur (RT) bzw. 50°C.

### Beispiel

Zur Evaluierung der Stabilität des Chemiesystems eines zur Bestimmung von Glucose dienenden diagnostischen Elements, umfassend Glucose-Dehydrogenase und Nikotinamid-Adenin-Dinukleotid, gegenüber ionisierender Strahlung wurden die Aktivität von Glucose-Dehydrogenase sowie der Gehalt an Nikotinamid-Adenin-Dinukleotid in dem diagnostischen Element vor und nach Sterilisation mit Elektronenstrahlung bzw. Gammastrahlung unter gleichzeitiger Variation der Parameter Lagerungsdauer und Lagerungstemperatur bestimmt.

Die Figuren 1 und 2 zeigen einen Vergleich der Aktivität von Glucose-Dehydrogenase (GlucDH) bzw. einen Vergleich des Gehalts an Nikotinamid-Adenin-Dinukleotid (NAD) in oben beschriebenem diagnostischen Element vor und nach Sterilisation mit Elektronenstrahlung. Wie aus den Figuren hervorgeht, wird die Aktivität von GlucDH bzw. der Gehalt an NAD durch Bestrahlung des diagnostischen Elements mit Elektronenstrahlung nicht beeinflusst.

Erstaunlicherweise liegen die Enzymaktivität und der NAD-Gehalt bei einem unmittelbar nach Herstellung des diagnostischen Elements (0 Wochen) durchgeführten Vergleich zwischen bestrahltem und nicht-bestrahltem diagnostischen Element jeweils bei 100%. Mit zunehmender Lagerungsdauer (0 bis 4 Wochen) und steigender Lagerungstemperatur (5°C bis 50°C) sinken sowohl die Enzymaktivität als auch der NAD-Gehalt in dem diagnostischen Element, unabhängig von einem eventuellen Einwirken sterilisierender Strahlung. Die vorherige Bestrahlung derart gelagerter diagnostischer Elemente bedingt jeweils eine geringfügige Abnahme der GlucDH-Aktivität bzw. des NAD-Gehalts, welche allerdings innerhalb der analytischen Fehlergrenzen liegt.

Die Figuren 3 und 4 zeigen einen Vergleich der Aktivität von Glucose-Dehydrogenase (GlucDH) bzw. einen Vergleich des Gehalts an Nikotinamid-Adenin-Dinukleotid (NAD) in oben beschriebenem diagnostischen Element vor und nach Sterilisation mit Gammastrahlung. Hierbei wird deutlich, dass sowohl das Enzym als auch das Coenzym selbst hochenergetische Gammastrahlung tolerieren, wenn auch in geringerem Ausmaß als bei Sterilisation mit Elektronenstrahlung.

Tatsächlich sinkt die Enzymaktivität in einem unmittelbar nach Herstellung sterilisierten System (0 Wochen) auf etwa 55% des ursprünglichen Werts ab, während die Schädigung bei Verwendung einer Kombination von Gammastrahlung und Kohlendioxid mit 80% enzymatischer Restaktivität geringer ausfällt. Im Falle des Coenzyms wird nach Sterilisation mit Gammastrahlung ein Restgehalt an NAD von etwa 90% detektiert; bei Verwendung einer Kombination von Gammastrahlung und Kohlendioxid ist kein Unterschied im NAD-Gehalt gegenüber einem unbehandeltem diagnostischen Element festzustellen.

Analog zu den mit Elektronenstrahlung durchgeführten Versuchen sinken die Enzymaktivität und der Gehalt an Coenzym mit zunehmender Lagerungsdauer (0 bis 4 Wochen) und steigender Lagerungstemperatur (5°C bis 50°C) sowohl in einem unbehandelten als auch in einem mit Gammastrahlung bzw. Gammastrahlung in Kombination mit Kohlendioxid behandelten diagnostischen Element, wobei die enzymatische Restaktivität und der Gehalt an NAD speziell im Falle der mit Gammastrahlung und Kohlendioxid behandelten diagnostischen Elemente die Werte der entsprechenden unbehandelten diagnostischen Elemente lediglich geringfügig unterschreitet.

## Patentansprüche

1. Diagnostisches Element, umfassend ein chemisches Nachweisreagenz, welches zumindest eine gegenüber ionisierender Strahlung empfindliche Komponente umfasst,
**dadurch gekennzeichnet,**
**dass** das diagnostische Element einer Sterilisation unterzogen worden ist und die gegenüber ionisierender Strahlung empfindliche Komponente nach Sterilisation zu einem Anteil von ≥ 80%, insbesondere zu einem Anteil von ≥ 90%, bezogen auf die Gesamtmenge der Komponente in dem diagnostischen Element vor der Sterilisation, in funktioneller Form vorliegt.

2. Diagnostisches Element nach Anspruch 1,
**dadurch gekennzeichnet,**
**dass** die gegenüber ionisierender Strahlung empfindliche Komponente ein Polypeptid, ein Coenzym, ein optischer Indikator, oder eine Kombination hiervon ist.

3. Diagnostisches Element nach Anspruch 2,
**dadurch gekennzeichnet,**
**dass** das Polypeptid ein Enzym, bevorzugt eine Nicotinamid-Adenin-Dinukleotid (NAD/NADH)- oder Nikotinamid-Adenin-Dinukleotidphosphat (NADP/NADPH)-abhängige Dehydrogenase, stärker bevorzugt eine Glucose-Dehydrogenase (EC 1.1.1.47) oder eine Glucose-6-phosphat-Dehydrogenase (EC 1.1.1.49), ist.

4. Diagnostisches Element nach Anspruch 2 oder 3,
**dadurch gekennzeichnet,**
**dass** das Coenzym eine NAD(P)/NAD(P)H-Verbindung, bevorzugt eine stabilisierte NAD(P)/NAD(P)H-Verbindung, stärker bevorzugt carbaNAD, ist.

5. Diagnostisches Element nach einem der Ansprüche 1-4,
**dadurch gekennzeichnet,**
**dass** es keinen Mediator umfasst.

6. Diagnostisches Element nach einem der Ansprüche 1-5,
**dadurch gekennzeichnet,**
**dass** es ein Probennahmeelement, insbesondere ein Nadelelement, integriert oder separat umfasst.

7. Diagnostisches Element nach Anspruch 6,
**dadurch gekennzeichnet,**
**dass** es zusammen mit dem Probennahmeelement in einem Vorratsbehälter, insbesondere in einem Magazin, gelagert ist.

8. Diagnostisches Element nach einem der Ansprüche 1-7,
**dadurch gekennzeichnet,**
**dass** es steril verpackt ist.

9. Diagnostisches Element nach einem der Ansprüche 1-8,
**dadurch gekennzeichnet,**
**dass** es zur Erzeugung eines optisch oder elektrochemisch detektierbaren Signals ausgebildet ist.

10. Diagnostisches Element nach einem der Ansprüche 1-9
**dadurch gekennzeichnet,**
**dass** es als Testelement mit integriertem Nadelelement, Testband oder Teststreifen ausgebildet ist.

11. Verfahren zur Herstellung eines diagnostischen Elements, umfassend die Schritte:
(a) Bereitstellen eines diagnostischen Elements, umfassend ein chemisches Nachweisreagenz, welches zumindest eine gegenüber ionisierender Strahlung empfindliche Komponente umfasst, wobei die mindestens eine gegenüber ionisierender Strahlung empfindliche Komponente eine Nikotinamid-Adenin-Dinukleotid (NAD/NADH)-oder Nikotinamid-Adenin-Dinukleotidphosphat (NADP/NADPH)-abhängige Dehydrogenase ist, und
(b) Sterilisieren des diagnostischen Elements mit ionisierender Strahlung, insbesondere mit Elektronenstrahlung oder/und Gammastrahlung.

12. Verfahren nach Anspruch 11,
**dadurch gekennzeichnet,**
**dass** das diagnostische Element weiterhin ein Probennahmeelement, insbesondere ein Nadelelement, integriert oder separat umfasst.

13. Verfahren nach Anspruch 11 oder 12,
**dadurch gekennzeichnet,**
**dass** es vor dem Sterilisieren das Einbringen des diagnostischen Elements in einen Vorratsbehälter, insbesondere in ein Magazin, umfasst.

14. Verfahren nach Anspruch 13,
**dadurch gekennzeichnet,**
**dass** es vor dem Sterilisieren weiterhin das Verschließen des Vorratsbehälters umfasst.

15. Verfahren nach einem Ansprüche 11 bis 14,
**dadurch gekennzeichnet,**
**dass** die Elektronenstrahlung eine Dosis im Bereich von 15-35 kGy, bevorzugt im Bereich von 20-30 kGy, aufweist bzw. die Gammastrahlung eine Dosis im Bereich von 15-35 kGy, bevorzugt im Bereich von 20-30 kGy, aufweist.
